# EUROPEAN PATENT APPLICATION

(11) **EP 2 377 572 A1**
(43) Date of publication of application: **19.10.2011**
(21) Application number: 11162772.5
(22) Date of filing: 18.04.2011
(51) Int. Cl.: A61N 1/05, A61N 1/372, A61B 5/04, G06N 3/06, G01N 33/487, A61N 1/36

(54) **A bio-hybrid implant for connecting a neural interface with a host nervous system**

(30) Priority: 19.04.2010 US 325744 P
(71) Applicant: IMEC, 3001 Leuven (BE); Katholieke Universiteit Leuven, K.U. Leuven R&D, 3000 Leuven (BE)
(72) Inventor: Huys, Roeland, 3012, Wilsele (BE); Braeken, Dries, 3900, Overpelt (BE); Prodanov, Dimiter, 1040, Etterbeek (BE); Eberle, Wolfgang, 3001, Leuven (BE); Verstreken, Kris, 2970, Schilde (BE)
(74) Representative: Hertoghe, Kris Angèle Louisa

(57) **Abstract**

A bio-hybrid implant suitable for recording and/or stimulating cells, the implant comprising
- at least one closed insulated chamber (1) containing a substrate (502) with a neural interface (13) for connecting neurons to an electronic circuit,
- at least one flexible guiding channel (10) having a first interface (11) to connect to at least one of the closed insulated chambers (1) and a second interface (9) to connect to a host's nerve system (7) or to another insulated chamber.

## Description

### Field of the invention

The present invention relates to the field of implantable (neuronal) devices, neuroprosthetics and cognitive prostheses. Furthermore the present invention relates to a device suitable for interfacing an electronic circuit with active cells (e.g. neurons) of the human body.

More particularly the present invention relates to biocompatible implantable devices, methods of manufacturing them and methods and systems of using such devices.

### Background of the invention

Interfacing an electronic device with neurons of the human body is crucial in the field of neuroprosthetics. Existing interfaces for connecting with a peripheral nerve are using electrodes that go in contact with the nerve system or living cells to be monitored. Examples of these electrodes are cuff-electrodes or sieve-electrodes or penetrating intraneural electrodes, while interfaces for connecting with the central nerve system are for example cylindrical electrodes for deep-brain stimulation, and multi electrode arrays, such as Utah-probe like systems, or Michigan-probe like systems. However these probe like systems encounter problems when a further advanced neuroprosthetic interface has to be realized, e.g. connecting a semiconductor device to the neural network. The major problems in this case are:
1. The ability to record neuronal signals with good Signal to Noise and Interference Ratio (SNIR) which requires that the electrodes need to be in close contact with the target neurons.
2. Damage to the target neurons and neurites due to a surgical procedure while implanting in the electrodes.
3. Long-term mechanical injury due to micro-motion of the probe relative to the brain.
4. Long-term alteration of the nervous tissue caused by neuro-inflammation (foreign-body reaction).
5. Reduction of the lifetime of the electrodes due to corrosion.
6. Lowering the power consumption of the implant, worsened by electrical stimulation of non-specific (large) targets, and low-SNR recording circuits resulting in complex data acquisition circuits and signal processing to interpret the signals.

To solve the above mentioned issues there is a need for a more efficient and reliable interface between the living tissue and the electronics.

### Summary of the invention

It is an object of embodiments of the present invention to provide an interface between electronic systems and neurons. In particular embodiments of the present invention, the interface may be made between a tissue, e.g. brain or cardiac tissue, on the one hand, and a neuro-computer on the other hand. Aspects of such interface, hence aspects of embodiments of the present invention are that the interface is
- capable of forming connections to an afferent nerve (e.g. sensory nerve for for example pain sensing), and/or
- capable of forming connections to an efferent nerve (e.g. motor nerve for for example controlling an artificial hand), and/or
- capable of forming connections to nuclei within the brain or the spinal cord, and/or
- capable of performing deep-brain stimulation (e.g. chronic implant for Parkinson's disease), and/or
- capable of forming a neural probe sensor array (e.g. for detecting epileptic seizure), and/or
- capable of forming a bi-directional brain interface (providing basis for so-called 'closed-loop' neural control systems).

It is an advantage of embodiments of the present invention that they solve problems related to existing interface systems such as cuff-electrodes, sieve-electrodes, Utah-probe like systems, or Michigan-probe like systems.

The problems related to existing interface systems are solved by a bio-hybrid implant according to embodiments of the present invention.

In a first aspect, the present invention provides a bio-hybrid implant suitable for recording and/or stimulating cells, the implant comprising at least one closed insulated chamber containing a substrate with a neural interface adapted for connecting neurons to an electronic circuit, and at least one flexible guiding channel having a first interface to connect to at least one of the insulated chambers and a second interface to connect to a host's nerve system or to another insulated chamber.

A bio-hybrid implant according to embodiments of the present invention solves problems related to existing interface systems by interfacing a hosts' neurons with naturally grown neurites. Said naturally grown neurites are guided in a closed system from/through a flexible guiding channel towards a neural interface, also called Neuronal Transducer Array (NTA) in the context of the present invention, which may for example be a MEA or any other suitable interface structure, and which may interface, e.g. make electrical connection, to an electronic circuit, e.g. in a chip. Said closed system is further referred to in general as the closed insulted chamber, and may further contain in-vitro cultured cells adhered to the neural interface, and a local fluidic circulation of support medium. The neurites are guided to and from the host body into the bio-hybrid implant via the flexible guiding channel. They may be recorded and/or stimulated by interfacing with the Neuronal Transducer Array (NTA). Alternatively, in-vitro cultured neurons can be stimulated to form neurites which are guided through the guiding channel to grow into the host body and making contact with the hosts' neurons.

Said bio-hybrid implant according to embodiments of the present invention further differs from existing interface systems in the fact that the electronic interface system (in this application referred to in general as the Neuronal Transducer Array (NTA)) may be situated outside the living tissue of interest (e.g. outside the brain or outside the cardiac tissue) or alternatively may be located within a non-critical tissue area (e.g. beneath skin). By letting in-vitro cultured neurites grow into the tissue of interest or alternatively let host's neurites grow outside its tissue it is possible that the actual connection with the Neuronal Transducer Array (NTA) can be made outside the tissue of interest in an insulated and controlled environment thereby significantly reducing the risk of contamination. The closed system can thereby efficiently separate the hosts' immune system from the cultured neurons, thereby reducing the risk of inflammation at the interface and reducing the risk of corrosion of the electrodes.

It is an advantage of at least some embodiments of the present invention that in-vitro cultured (dissociated) neurons adhere excellently to a planar interface, especially if the interface is functionalized and/or has an optimized micro-topology.

It is a further advantage of at least some embodiments of the present invention that thanks to the closed system in-vitro cultured neurons can be separated (isolated) from host's neurons by a guiding channel. This makes it possible that in-vitro cultured neurons can be selected from a wide range of cells originating from stem cells, primary cell cultures, in-vitro genetically transfected cells from genetically modified embryos of organisms, etc... Thanks to the closed system optionally having an integrated fluidic system, the cells can have their own support of culture medium or other biochemical compounds. The culturing process can thereby be performed in controlled conditions to optimize the result.

It is a yet further advantage of at least some embodiments of the present invention that the substrate comprising the Neuronal Transducer Array (NTA), or the NTA itself, may further comprise micro-nail electrodes, as for example described in EP 1967581, which allow for a large numbers of neurons to be addressed with a more efficient neuron-sensor coupling. The micro-nail electrodes may be substantially perpendicular to the plane of the substrate. Said micro-nail electrodes can further comprise in-situ CMOS circuits, special micro-structures or special micro-biochemical structures, such as patterns of deposited chemicals (chemical patterning), patterned surface micro-structures, bio-sensors, micro-fluidic devices, optical sensors, MEM's like devices, etc....

It is further an advantage of embodiments of the present invention that the bio-hybrid implantable system of the current invention is directly addressable thereby making it possible to stimulate large populations of neurons thanks to power-efficient single-cell stimulation. Furthermore said direct addressability is leading towards a significant increased Signal To Noise Ratio (SNR) of the recorded signal read-out thereby making it possible to use low-power acquisition circuits and less complex signal processing algorithms.

It is further an advantage of embodiments of the present invention that the bio-hybrid implantable system of the current invention is capable of addressing a high number of cells leading to a significant increase in density read out.

It is further an advantage of embodiments of the present invention that additional supporting cells can be adhered to the NTA of the bio-hybrid implantable system. The use of different cells has the advantage that different/specific neurons may be addressed and/or contacted by the bio-hybrid implant.

It is an advantage of embodiments of the present invention that the guiding channel can be made of an elastic material which can be stretched or shortened depending on the needs, thereby giving a great flexibility towards possible ways of implanting the system onto/into a human body e.g. sub-dermal. Further details on the properties and ways how to manufacture the guiding channels can be found in IEEE TRANSACTIONS ON NEURAL SYSTEMS AND REHABILITATION ENGINEERING, VOL. 17, NO. 5, OCTOBER 2009, Long Micro-Channel Electrode Arrays: A Novel Type of Regenerative Peripheral Nerve Interface, Stéphanie P. Lacour.

The insulated chamber of a bio-hybrid implant according to embodiments of the present invention may comprise at least one of an electronic circuit, a chip, a biosensor, an optical sensor, an optical stimulator, a chemical sensor or a chemical stimulator.

The substrate in the insulated chamber of the implant may be made of or comprise any of silicon, glass, SOI, and/or polymer material.

The at least one closed insulated chamber of the implant may further comprise in-vitro cultured neuron cells and optionally further supporting cells such as Schwann cells, oligodendrocytes or other glial cells. It is an advantage of embodiments of the present invention that the addition of glial cells in the culture medium can improve long-term stability because of its supporting function for neurons.

The at least one closed insulated chamber of the implant may further comprise electronic devices for interfacing with the at least one guiding channel. The at least one closed insulated chamber may furthermore comprise micro-fluidic devices for viability of the cells. It is an advantage of embodiments of the present invention that the addition of micro-fluidic apparatus such as micro-fluidic channels make it possible to deliver supporting media & growth factors, deliver supporting cells such as glial cells, and to provide a dialysis system for the hosts' body.

In accordance with embodiments of the present invention, the substrate may further comprise micro-nail electrodes. The micro-nail electrodes may be substantially perpendicular to the plane of the substrate. Optionally, the micro-nail electrodes may comprise in-situ CMOS circuits, special micro-structures or bio-structures such as patterns of deposited chemicals, patterned surface micro-structures, bio-sensors, micro-fluidic devices, optical sensors, MEM's like devices, etc..... It is an advantage of embodiments of the present invention that due to the presence of additional micro-needle structures to the substrate a larger numbers of neurons can be addressed with a more efficient neuron-sensor coupling. It is a further advantage of embodiments of the present invention that the additional micro-needle structures make it possible to adhere (in-vitro) cells onto said micro-needle structures and make it possible to guide cells or in other words cells will grow into a predefined direction defined by the micro-needle structures.

The guiding channel(s) of the implant may be made of a bio-compatible, preferably flexible and optionally stretchable material having mechanical compatibility with the host's tissues. A guiding channel may be able to protect the axons, in particular where they would be passing through regions of strong mechanical strain exerted e.g. by surrounding tissue. It is an advantage of embodiments according to the present invention that a guiding channel (made of a stretchable material) may be shortened depending on demand material. It is a further advantage of at least some embodiments of the present invention that a guiding channel made of an elastic material, which can be stretched or shortened, is giving a great flexibility towards implantation of the system onto the human body e.g. sub-dermal.

The at least one guiding channel of the implant may be made of oxides, silicon, silicone, polymers (such as polyimide, PDMS,..) and/or thin metals.

The at least one guiding channels, in particular a plurality of guiding channels, of the implant may be bundled and surrounded by a further flexible tube. Also such further flexible tube may be made of oxides, silicon, silicone , polymers (such as polyimide, PDMS,..) and/or thin metals.

The guiding channel(s) of the implant may have a longitudinal surface, and may further comprise electrodes and/or openings along its longitudinal surface. It is an advantage of embodiments of the present invention that openings in the guiding channel can be used to let neurites grow through and make synaptic connections with a peripheral or a spinal nerve. Said openings may in particular be made such that (i) the culture medium does not leak outside the channel and/or (ii) they can achieve selective axonal outgrowth through the openings (meaning that certain diameter ranges block the outgrowth of certain types of axons and allow passage of others). The diameter of said openings may be in the range of 10µm up to 50 µm. It is a further advantage of embodiments of the present invention that electrodes can be provided on the inner surface of the at least one guiding tube. Said electrodes can be used e.g. for detecting how far neurites have grown into the guiding channel (detecting progress). It is a further advantage of embodiments of the present invention that electrodes can be provided on the outer surface of the at least one guiding channel. Said electrodes can be used e.g. for deep brain stimulation.

According to particular embodiments of the present invention, the implant may be used for controlling and/or observing a host's nervous system which can be achieved by contacting in vitro cultured neurons of the implant with a host's nervous system. In a first alternative, the in vitro neuron cells may be used to record a host's neuronal activity (observation). In a second alternative the in vitro neuron cells of the implant may be used to control a host's neuron activity by selectively stimulating certain populations of neurons (control). In a third alternative the in vitro neuron cells of the implant may be used to first control and then record a host's neuron activity by first stimulating (e.g. by evoking a potential) the host's nervous system and then observing the host's neuron activity. In a fourth alternative the in vitro neuron cells of the implant may be used to first record the host's neuron activity and then control a host's nervous system by first observing and then stimulating the host's neuron activity (e.g. by evoking a potential) and repeating these steps (if necessary) in a closed control loop.

According to a second aspect, the present invention relates to a method for manufacturing a bio-hybrid implant, the method comprising at least steps of:
- obtaining a supporting substrate,
- providing on said substrate a neural interface for connecting neurons to an electronic circuit,
- providing a packaging around said substrate and neural interface to obtain a closed chamber which is isolated from an outside environment,
- providing at least one flexible guiding channel going into said closed chamber and making contact to said neural interface, and
- providing means to promote growth of neurites into the inner surface of said at least one guiding channel.

It is an advantage of at least some embodiments of the present invention that conventional processing steps can be used for manufacturing the different components of the bio-hybrid implant.

The method for manufacturing the implant according to embodiments of the present invention may further comprise the step of providing at least one of an electronic circuit, a chip, a biosensor, an optical sensor, an optical stimulator, a chemical sensor or a chemical stimulator and the method may furthermore comprise the step of providing further electronic devices for interfacing with the at least one guiding channel and/or providing micro-fluidic devices for viability of the cells.

The method for manufacturing the implant according to embodiments of the present invention may further comprise the step of providing in-vitro cultured neuron cells and/or supporting cells attached to the substrate.

Particular and preferred aspects of the invention are set out in the accompanying independent and dependent claims. Features from the dependent claims may be combined with features of the independent claims and with features of other dependent claims as appropriate and not merely as explicitly set out in the claims.

These and other aspects of the invention will be apparent from and elucidated with reference to the embodiment(s) described hereinafter.

### Brief description of the drawings

Figure 1 illustrates a schematic representation of a bio-hybrid implant for connecting a host's efferent neurons with a neural interface according to an embodiment of the present invention.
Figure 2 illustrates a schematic representation of a bio-hybrid implant for connecting in-vitro cultured neurons with afferent (e.g. sensory) neurons of a host, according to an embodiment of the present invention.
Figure 3 illustrates a schematic representation of a bio-hybrid implant for connecting in-vitro cultured neurons with myelated axons according to an embodiment of the present invention.
Figure 4 illustrates a schematic representation of a bio-hybrid implant for connecting in-vitro cultured neurons with a host central nervous system according to an embodiment of the present invention.
Figure 5 illustrates a schematic representation of two bio-hybrid systems connected together, forming an in-vitro test set-up for evaluating the interconnectivity of two different cell cultures, or of a cell culture with a tissue slice such as for example a brain slice according to an embodiment of the present invention.
Figure 6 illustrates a schematic representation of a dorsal connection method for the bio-hybrid implant according to an embodiment of the present invention.
Figure 7 illustrates a schematic representation of a vertical axon connection method for the bio-hybrid implant according to an embodiment of the present invention.
Figure 8 illustrates a schematic representation of a lateral axon connection method for the bio-hybrid implant according to an embodiment of the present invention.
Figure 9 illustrates a schematic representation of an implementation of a sub-dermal bio-hybrid implant according to an embodiment of the present invention.
Figure 10 illustrates a first Unidirectional System, corresponding to the bio-hybrid implant and its use as set out with respect to Figure 1.
Figure 11 illustrates a second Unidirectional System, corresponding to the bio-hybrid implant and its use as set out with respect to Figure 2.
Figure 12 illustrates a bio-hybrid system comprising both a first Unidirectional System as illustrated in Figure 10 and a second Unidirectional System as illustrated in Figure 11, and a central signal interpretation and decision part for electrically connecting both Unidirectional Systems.

The drawings are only schematic and are non-limiting. In the drawings, the size of some of the elements may be exaggerated and not drawn on scale for illustrative purposes.

Any reference signs in the claims shall not be construed as limiting the scope.

In the different drawings, the same reference signs refer to the same or analogous elements.

### Detailed description of illustrative embodiments

As used herein and unless stated otherwise, neurons or nerve cells are electrically excitable cells that process and transmit information by electrical and chemical signalling.

In this context, the term "neurites" relates to any projection from the cell body of a neuron. This projection can be either an axon or a dendrite. Dendrites are filaments that arise from the cell body, often extending for hundreds of micrometers and branching multiple times. Dendrites are responsible for receiving signals and conducting them up the cell to the cell body and on to the axon. An axon is a special cellular filament that arises from the cell body and travels for a distance, as far as 1m in humans or even more in other species. The axon is the portion of the neuron that is responsible for passing of the cellular message from the neuron to either other neurons or to neural receptors, e.g. targets of nerve impulses such as muscles. The cell body of a neuron frequently gives rise to multiple dendrites, but never to more than one axon, although the axon may branch a plurality of times before it terminates. The term "neurites" is frequently used when speaking of immature or developing neurons, especially of cells in culture, because it can be difficult to differentiate axons from dendrites before the differentiation is complete.

Efferent neurons, also known as motor or effector neurons, carry nerve impulses away from the central nervous system to effectors such as muscles or glands. The opposite activity or direction or flow is afferent. Afferent neurons, also known as sensory or receptor neurons, carry nerve impulses from receptor or sense organs towards the central nervous system.

A synapse is a junction that permits a neuron to pass an electrical or chemical signal to another cell (neural or otherwise). At a synapse, the plasma membrane of the signal-passing neuron (the presynaptic neuron) comes into close apposition with the membrane of the target (postsynaptic) cell. Both the presynaptic and postsynaptic sites contain extensive arrays of molecular machinery that link the two membranes together and carry out the signalling process.

As used herein and unless stated otherwise, the term "Neuronal Transducer Array" or "NTA" relates to any suitable neural interface for connecting neurons to electronic circuitry. The NTA may for example include any suitable electronic circuit, such as any suitable chip. The NTA may comprise any suitable Multi Electrode Array (MEA). Said NTA may further comprise one or more of bio-sensors, chemical stimulators, optical sensors/stimulators, patterned structures such as micro-nails,...

Multielectrode arrays (MEAs) or microelectrode arrays are devices that contain multiple plates or shanks through which neural signals are obtained or delivered, essentially serving as neural interfaces that connect neurons to electronic circuitry. There are two general classes of MEAs: implantable MEAs for use in vivo, and non-implantable MEAs for use in vitro.

The invention will now be described by a detailed description of several embodiments thereof. It is clear that other embodiments of the invention can be configured according to the knowledge of persons skilled in the art without departing from the technical teaching of the invention, the invention being limited only by the terms of the appended claims.

By way of illustration, embodiments of the present invention not being limited thereto, a more detailed description of features and advantages of at least some embodiments of the present invention will be described with respect to the figures which are used to illustrate the embodiments but which are not intended to be limiting for the invention.

According to a first aspect of the invention a "bio-hybrid implant" is disclosed. Such bio-hybrid implant according to the first aspect is a device which is suitable for realizing an interface between neurons, e.g. a living hosts' neurons (e.g. by naturally grown neurites), on the one hand, and electronics on the other hand. A "bio-hybrid implant" according to embodiments of the present invention may be used to stimulate and/or record the host's neuron activity. The "bio-hybrid implant" is preferably a closed system containing a neural interface with optionally added in-vitro cultured cells and a local fluid circulation of supporting medium. The interface itself between the host's neurons and the electronics is preferably achieved by guiding neurites to and/or from the host body through a flexible guiding channel. The goal of the bio-hybrid implant of embodiments of the current invention may be stimulation and/or recording signals from living neuron cells.

A bio-hybrid implant according to the first aspect of the present invention comprises a closed insulated system containing a substrate with a neural interface, also called Neuronal Transducer Array (NTA) in the context of the present invention, adapted for connecting neurons to an electronic circuit, and at least one flexible guiding channel having a first interface to connect to the closed insulated system and a second interface to connect to a host's nerve system. The adaptation of the neural interface is such that growth and guidance of neurons on and over the interface is promoted, for example by functionalizing and/or optimizing micro-topology of the interface surface. This may be done by coating or texturing the surface of the neural interface where neurons grow, such as for example, but not limited thereto, by providing grooves in the surface of the neural interface.

According to a first embodiment of the first aspect, the present invention relates to a bio-hybrid implant for guiding and connecting a host's efferent neurons, also known as motor or effector neurons, towards an insulated system containing the Neuronal Transducer Array. The insulated system may preferably furthermore contain in-vitro cultured neurons.

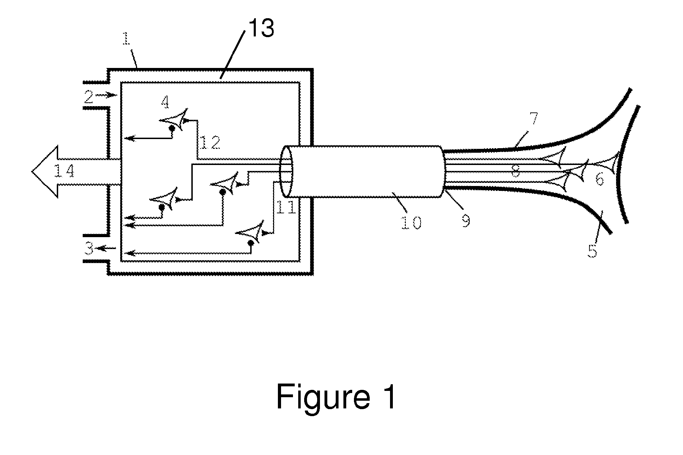

Figure 1 illustrates a schematic representation of such bio-hybrid implant according to the first embodiment of the first aspect of the present invention, connecting to an efferent (e.g. motor) nerve 7. Three main blocks can be seen in this bio-hybrid system. A first block comprises an insulated chamber 1 containing the Neuronal Transducer Array 13, for example a MEA. In the embodiment illustrated, the insulated chamber 1 furthermore comprises electronic devices for interfacing, and micro-fluidic apparatus for providing viability of the cells. A second block comprises at least one flexible guiding channel 10, optionally bundled in a further flexible tube (not illustrated in Figure 1). A third block comprises the hosts' nervous system (neurons), in particular in the embodiment illustrated the efferent nerve 7, making contact via the guiding channel 10 with the neural interface 13. The neurons of interest in the efferent nerve 7 may be e.g. motor neurons 6 located in the ventral horn of the spinal cord 5 of the host, for example to connect the neuronal prosthesis in the form of the bio-hybrid implant in accordance with embodiments of the present invention with an interrupted nerve 7 after amputation e.g. for controlling an artificial limb. The axons 8 of the motor neurons 6 can be stimulated to regenerate, and they can be guided through the flexible guiding channel 10. The interface 9 of the guiding channel 10 to connect to the nerve 7 should comprise a means to promote neurite outgrowth and guiding, and should be made of bio-compatible materials. The guiding channel 10 is connected to the insulated chamber 1 with an interface 11 which ensures outgrowth of neurites 12 and guiding thereof on the surface of the neural interface 13 such that the grown neurites 12 can make connection to in-vitro cultured neurons 4 present in the insulated chamber 1. The way these connections are made, can be controlled by chemical guiding, by patterning of surface chemistry, topological cues, electrical interactions, etc...

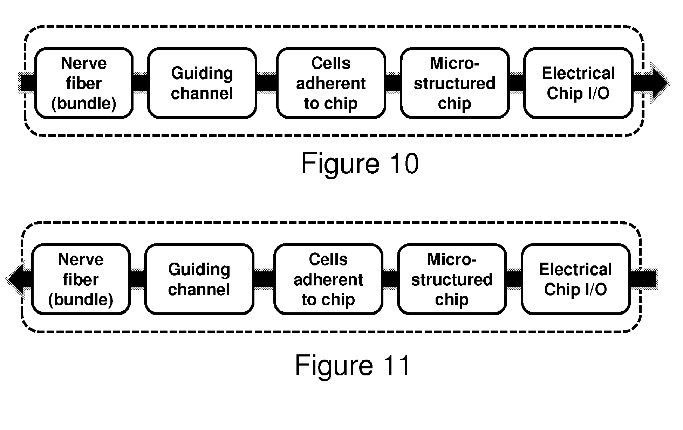

The insulated chamber 1 may further contain an inlet 2 and an outlet 3 of a fluidic, e.g. microfluidic, system, providing the in vitro culture medium with oxygen, growth factors, and other (bio) chemical support as needed. This way, the chamber 1 can function as a perfusion chamber. It may be constructed in such a way that the in vitro cells will not be harmed e.g. by a strong flow of liquid (shear stress). Hence in particular direct flow over the in vitro cultured cells will be prevented. Perfusion in and outside the chamber 1 can be controlled by any suitable means, for example a micropump (not illustrated) which can be mounted under the skin and is connected to the (micro)fluidic system in the chamber 1. The (micro)fluidic system can contain biochemical molecules such as Netrins, Ephrin B or growth promotors such as NGF, BDNF, GDNF to attract growth of the neurites 12 towards the Neuronal Transducer Array 13. Suitable microfluidic systems which may be implemented in bio-hybrid implants according to embodiments of the present invention are described by Thomas Stieglitz in "Integration of Microfluidic Capabilities into Micromachined Neural Implants", International Journal of Micro-nano Scale Transport, Vol.1 1 (2), 2010.Figure 1 shows, by means of the arrow indicated by reference number 14, the information flow of cellular signals which, in accordance with the first embodiment of the first aspect, are set from tissue or cells to an outside system, such as e.g. an integrated computer or a wired or wireless data interface, and are acquired by the electronic circuit, e.g. sensors and circuits on a chip in the insulated chamber 1. The above first embodiment of the first aspect leads to a unidirectional system as further illustrated in Figure 10. The black arrow indicates a signal direction: biological signals are recorded and read out. Hereto, the nerve fibre 7, or more particularly axons 8 of e.g. motor neurons 6 thereof, are led to and grown through a guiding channel 10 of the bio-hybrid implant. These axons connect to in vitro cultured cells which adhere to the neural interface 13 in the insulated chamber 1. The neural interface 13 provides an electrical connection between the axons and input/output ports of an electrical circuit, e.g. a chip, present in the insulated chamber 1.

This unidirectional system is further called "Unidirectional System A".

According to a second embodiment of the first aspect, the present invention relates to a bio-hybrid implant for guiding in-vitro cultured neurons through the guiding channel towards the host's nervous system thereby making contact to e.g. an afferent nerve, also known as sensory or receptor nerve.

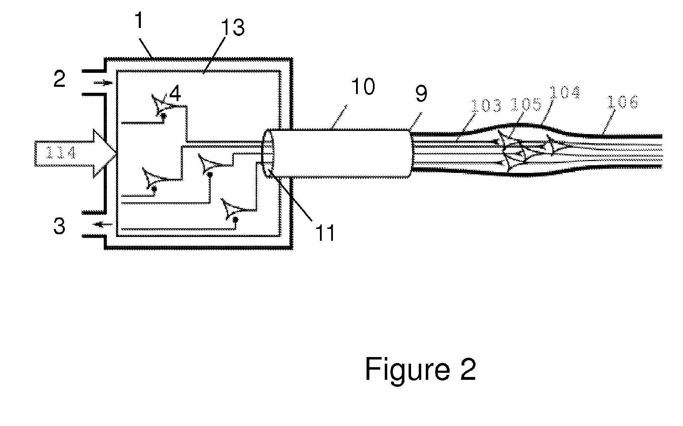

A schematic representation of a bio-hybrid implant according to this embodiment is shown in Figure 2. The bio-hybrid implant comprises an insulated chamber 1 containing the Neuronal Transducer Array 13, for example a MEA. In the embodiment illustrated, the insulated chamber 1 furthermore comprises in vitro cultured neurons 4, micro-fluidic apparatus for providing viability of the in vitro cultured neurons 4 and electronic devices for interfacing with the in vitro cultured neurons. The insulated chamber 1 may further contain an inlet 2 and an outlet 3 of a fluidic, e.g. microfluidic, system, providing the in vitro culture medium with oxygen, growth factors, and other (bio) chemical support as needed. This way, the chamber 1 can function as a perfusion chamber, as also explained with reference to Figure 1.

The axons 103 of the in vitro cultured neurons 4 can be stimulated to regenerate, and they can be guided through at least one flexible guiding channel 10, optionally bundled in a further flexible tube (not illustrated in Figure 2), connected to the insulated chamber 1. The interface 1 of the guiding channel 10 to connect to the chamber 1 should comprise a means to promote neurite growth and guiding, and should be made of bio-compatible materials. The guiding channel 10 is provided with an interface 9 which ensures outgrowth of neurites, for example axons 103, and guiding thereof towards the afferent nerve such that the axons 103 grown from in vitro cultured neurons 4 can make connection to an afferent nerve of the host's nervous system.

In this example, neurons 105 e.g. from the dorsal root ganglion 104 form synapses with the grown axons 103 from the cultured neurons 4 onto the NTA 13 and they detect signals coming along a (regenerated) afferent nerve. They send sensory signals through axons 106 to other neurons in the spinal cord, e.g. for triggering a motor reflex. The system is identical to the first described embodiment and as shown in Figure 1, except that the signal direction is different, in the sense that the major function of the chip will be stimulation (generation of action potentials) of the cells.

Figure 2 shows, by means of the arrow indicated by reference number 114, the information flow of cellular signals which, in accordance with the second embodiment of the first aspect, come from an outside system, such as e.g. an integrated computer, or a wired or wireless data interface, providing information such as e.g. patterns or waveforms, to stimulate cells. The above second embodiment of the first aspect leads to a unidirectional system as further illustrated in Figure 11. The black arrow indicates a signal direction: electrical signals are passed on to stimulate biological fibres. Hereto, electrical signals are provided, via input/output ports of an electrical circuit, e.g. a chip, present in the insulated chamber 1. These signals pass via the neural interface 13 and via in vitro cultured cells which adhere to the neural interface 13 in the insulated chamber 1. These in vitro cultured cells are stimulated to generate axons 103, which are led to and through the guiding channel 103, so as to make a connection to the nerve fibre. Electrical signals emanating from the electronics may be used to stimulate the nerve fibre.

This unidirectional system is further called "Unidirectional System A"'.

It can be noticed that this Unidirectional System A' is more difficult to implement than Unidirectional System A.

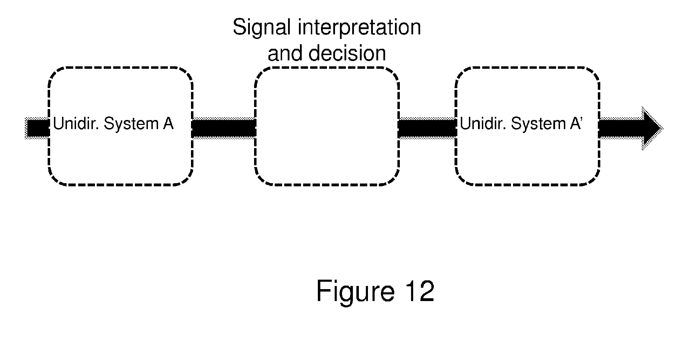

In embodiments of the present invention, as also illustrated in Figure 12, both Unidirectional System A and Unidirectional System A' may be used in a single in vivo to in vivo system. This may for example be implemented if a nerve would be interrupted, for example cut during an accident. In principle, both nerve ends could be grown together, optionally over a bio-hybrid implant according to embodiments of the present invention. However, when doing so, the problem arises that one cannot be sure that in the nerve bundle right nerve fibres connect to one another. In order to solve this problem, in accordance with embodiments of the present invention, one nerve end can be connected to a first bio-hybrid implant in accordance with embodiments of the present invention, so as to form Unidirectional System A, while the other nerve end can be connected to a second bio-hybrid implant in accordance with embodiments of the present invention, so as to form Unidirectional System A'. Measurements can be performed on both Unidirectional System A and on Unidirectional System A', so as to know which locations on the neural interfaces 13 in both Unidirectional Systems correspond to which fibres in the nerve bundle. Once this assessment has been made, a central electrical connection can be made between both neural interfaces 13. This central electrical connection can be external to, or can be implanted into a living host. This central electrical connection can be built based on conventional electronics or microelectronics, and is therefore not considered here in more detail.

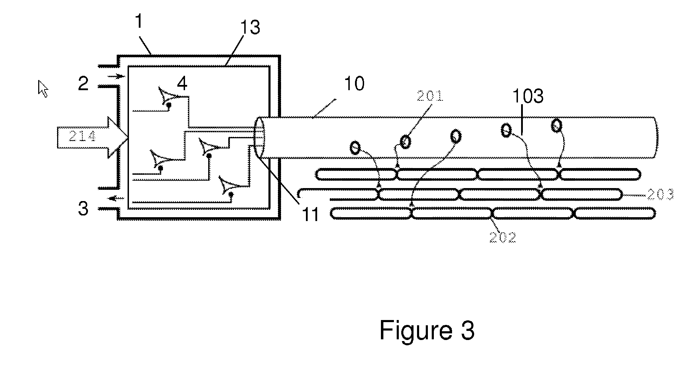

According to a third embodiment of the first aspect, the present invention relates to a bio-hybrid implant as shown in Figure 3, where axons 103 originating from in-vitro cultured cells 4 are guided through at least one guiding channel 10, and make synaptic connections with the nodes of Ranvier 202 of myelated axons 203 in a peripheral nerve system (PNS) or Central Nerve system (CNS). Hereto, the at least one guiding channel 10 may be provided with openings 103 along its longitudinal surface, the openings 103 being arranged for letting the axons 103 originating from the in vitro cultured cells 4 leave the at least one guiding channel 10. Figure 3 furthermore shows, by means of the arrow indicated by reference number 214, the information flow of cellular signals which, in accordance with the third embodiment of the first aspect, come from an outside system, such as e.g. an integrated computer, or a wired or wireless data interface, providing information such as e.g. patterns or waveforms, to stimulate cells.

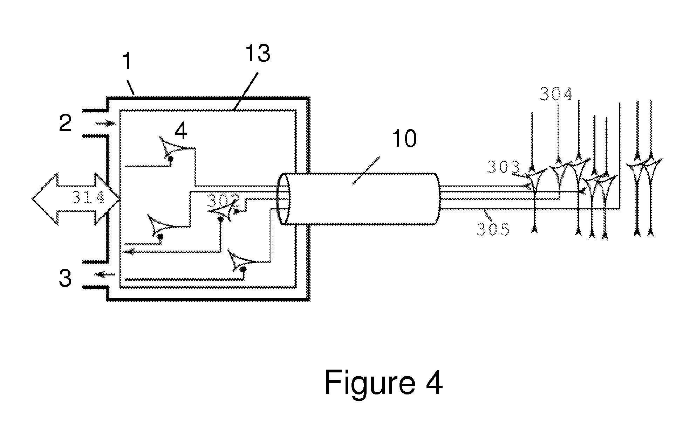

According to a fourth embodiment of the first aspect, the present invention relates to a bio-hybrid implant suitable for interfacing with the central nervous system (CNS), for example with a part of the brain cortex as shown in Figure 4. This illustrates that an implant according to embodiments of the present invention can have a bi-directional interface. Figure 4 illustrates, by means of the arrow indicated by reference number 314, the bi-directional information flow going to or coming from an outside system such as e.g. an integrated computer, or a wired or wireless data interface, with information to or from the cells, e.g. patterns, waveforms or recorded signals. This information may be acquired from and/or sent to the electronic circuit in the insulated chamber 1, e.g. transducers and circuit integrated on the chip. This bi-directional interface is obtained in this embodiment by guiding the neurites 302 of the in-vitro cultured cells 4 through the at least one guiding channel 10 towards neurons in the tissue area of interest, e.g. the brain area or the cardiac area. These neurons can form synapses to cells in this area of interest, functioning either excitatory or inhibitory, depending on factors like the cells being cultured, differentiation factors, chemical cues. The bi-directional interface is obtained by also guiding axons 304 and 305 originating from the CNS towards the in vitro cultured neurons 4. The interfacing of the signals may be achieved by reading directly the neurites coming from the host by the interface of the axon with an electrode, or indirectly by a synaptic connection with a cultured neuron 4. In the latter case, different axons might connect (project) signals to a population of cell bodies. The latter case, and other cases where complicated interfacing is required, would require analysis and interaction with a neuronal network, and possibly a bidirectional learning interface: the computer system has to learn the behavior and plasticity of the neuronal interface, and vice versa, the neurons can be trained to respond to specific patterns by applying different stimuli which influence the strength of synaptic connections. Other, more advanced methods might also be possible.

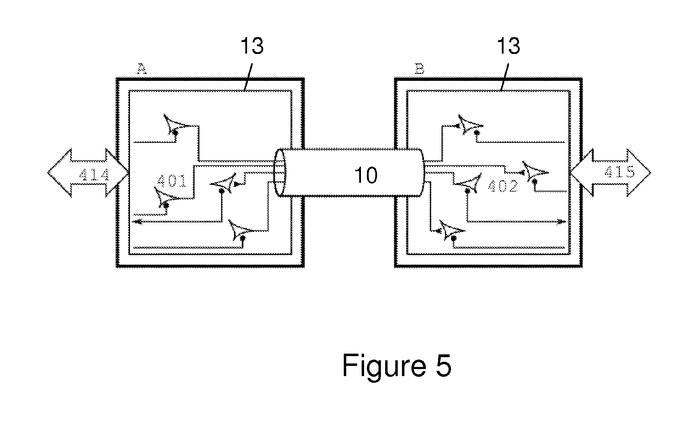

According to a fifth embodiment of the first aspect, the present invention relates to a method to apply the concept of the bio-hybrid implant in accordance with embodiments of the present invention in a more generic way. Preferably at least two insulated closed chambers A, B are provided containing a neural interface 13 for connecting neurons to an electronic circuit, for example a MEA. In the embodiment illustrated, the insulated chambers A, B furthermore comprise electronic devices for interfacing, e.g. a chip, and microfluidic apparatus for providing viability of cells. The chambers A, B may furthermore contain in-vitro cultured neurons 401, 402. In the embodiment illustrated, these in vitro cultured neurons 401, 402 are connected to each other with at least one guiding channel 10, thereby connecting the neurons of both insulated chambers A, B in a similar way as described in embodiments above. This way two different cell cultures, or even combinations of cell cultures e.g. in form of tissue slices such as brain slices or cardiac slices, can be used. This generic system is suitable as test module to perform in-vitro experiments e.g. to test effects of pharmaceutical products on neuron physiology,... Both insulated closed chambers A, B may further contain an inlet and an outlet for a fluidic system (not illustrated in Figure 5), providing the culture medium with oxygen, growth factors, and other (bio) chemical support as required.

Figure 5 illustrates, by means of the arrows indicated by reference numbers 414 and 415, the bi-directional information flows going to or coming from an outside system at either side of the assembly, such as e.g. an integrated computer, or a wired or wireless data interface, with information to or from the cells, e.g. patterns, waveforms or recorded signals. This information may be acquired from and/or sent to the electronic circuit in the insulated chamber 1, e.g. transducers and circuit integrated on the chip.

According to the embodiments described above a flexible guiding channel 10 is used to connect the at least one insulated chamber 1, A, comprising a neural interface 13 such as a MEA, and optionally electronic circuitry such as e.g. a chip , and which preferably furthermore contains in-vitro cultured neurons 4, 401 with a hosts' nerve system (more particularly neurons thereof) or with another insulated chamber B, also comprising in vitro cultured neurons 402.

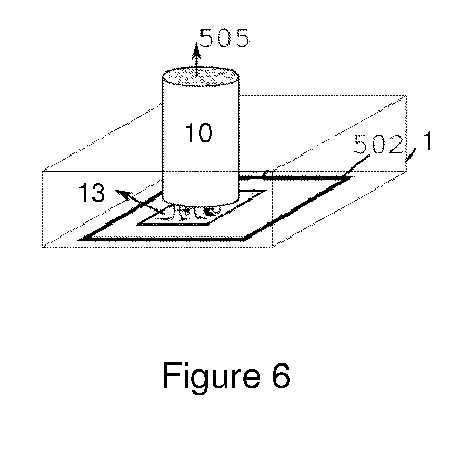

According to a first particular option, said at least one flexible guiding channel 10 may be applied according to a 'dorsal connection method' as shown in Figure 6, wherein the guiding channel 10 is brought vertical to the substrate 502 of the closed insulated chamber 1 of the bio-hybrid implant, thereby covering most of the surface of the NTA 13. The guiding channel 10 can further contain artificial micro-tubuli or micro-fibres 505 which promote the growth and guiding of axons 12, 103.

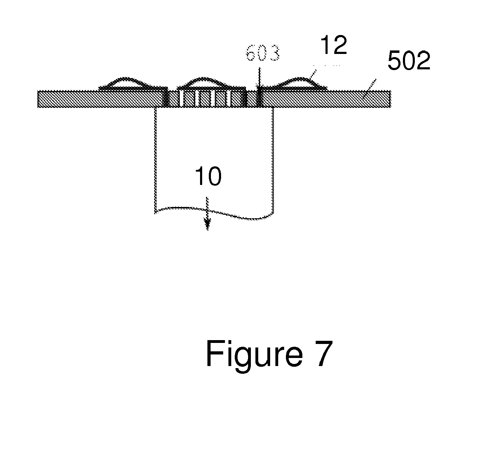

According to a second particular option, the at least one flexible guiding channel 10 may be applied upside-down as shown in Figure 7, which may for example be realized by making holes 603 in the substrate 502 of the closed insulated chamber 1 and guiding the neurites 12 downward through the surface of the NTA 13.

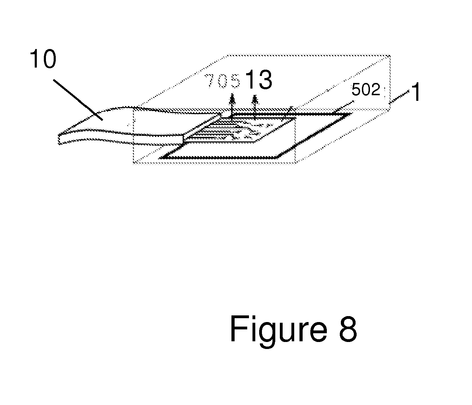

According to yet another particular (third) option, said at least one flexible guiding channel 10 is applied lateral at the side of the NTA 13, as shown in Figure 8. In this case, the at least one guiding channel 10 is a flat guiding channel (rather than a tubular one) which gives advantages with respect to integration with an electronic system such as a chip. However, in accordance with embodiments of the present invention (not illustrated), other possibilities like multi-layer (sandwich-like) or spirally-folded flat cable approaches are also possible. The surface of the NTA can furthermore contain means 705 for guiding the neurites 12 to the guiding channel 10, e.g. by topological cues, patterning of surface chemistry, etc...

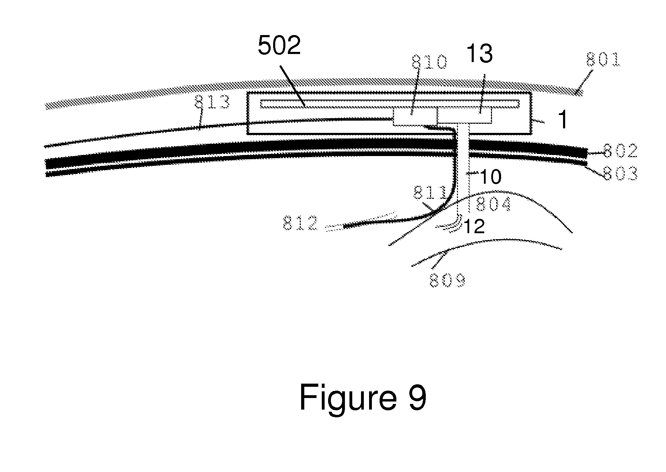

According to a sixth embodiment of the first aspect, the present invention relates to a bio-hybrid implant suitable for use as a sub-dermal implant for interfacing with the CNS as is shown in Figure 9. At least one closed insulated chamber 1 is thereby preferably implanted underneath the skin 801. The insulated chamber 1 contains a substrate with a neural interface 13 for connecting neurons to an electronic circuit, e.g. chip, the electronic circuit and all necessary power supply, data link and support circuits to connect to the neural interface 13 to the electronic circuit. Optionally said sub-dermal implant furthermore contains a micro-fluidic system 810 containing pumps, filters and possibly a storage of chemicals. The at least one flexible guiding channel 10 is preferably implanted sub-dermal, where micro-tubuli 505 originating from the guiding channel 10 spread out and guide the neurites 12, which spread further in the tissue, e.g. cortex, and make connections. Figure 9 illustrates as an example the implementation of a sub-dermal bio-hybrid implant. The insulated chamber 1 of the bio-hybrid implant containing the Neuronal Transducer Array 13 and (optional) in-vitro cultured neurons is implanted underneath the patient skin 801, just above the skull 802 and dura mater 803. The guiding channel 10 containing many micro-sized guiding channels 505 is further implanted through the skull 802 and dura mater 803 to make contact to the tissue region of interest, in the example illustrated the brain region of interest, for example a cortical area (804) connecting neurites 12 to a three-dimensional volume of brain tissue 809. Furthermore, the bio-hybrid implant according to this embodiment may comprise a micro-fluidic dialysis system 810 containing micro-pumps and filters, providing culture medium for the cell cultures and a flexible tube 811 for extracting celebro-spinal fluid (CSF) from the lateral ventricle for extracting CSF. An outlet tube 813 may be provided for drainage of waste CSF into a peritoneal cavity. This outlet tube 813 is a tube which may be connected at one end to the micro-fluidic system of the device. At the other end this tube can go to a lateral ventricle.

In a second aspect, the present invention relates to a method for manufacturing a bio-hybrid implant suitable for realizing an interface between neurons, e.g. living hosts' neurons (e.g. by naturally grown neurites) on the one hand, and electronics such as for example a chip, on the other hand. The implant comprises at least one closed insulated chamber 1, with optional adhered in-vitro cultured cells and local fluidic circulation of support medium. The interface itself is preferably made by guiding neurites to and/or from the host body (being a living host or an in vitro cultured host) by at least one flexible guiding channel 10. The method may be especially suitable for making a bio-hybrid implant used to stimulate and/or record hosts' neuron signals. The method according to embodiments of the present invention comprises in a first step obtaining a substrate 502, for example made of a bio-compatible material such as silicon, silicone and/or polymers, and providing a neural interface 13 into/onto said substrate 502. Said neural interface 13 may be a MEA. The neural interface 13 may be electrically connected an electronic circuit, such as for example a chip. The neural interface 13 and the substrate 502 are then packed into a biocompatible package to achieve a closed chamber 1 which is isolated from the outside environment. Said biocompatible package may be made out of biocompatible materials such as polymers PDMS, parylene^{®},... The method according to embodiments of the present invention further comprises the step of providing at least one guiding channel 10 going into said closed chamber 1 and having a first surface (11) to connect to the neural interface 13. If said at least one guiding channels comprises more than one guiding channel 10, the plurality of guiding channels 10 may be bundled together in at least one flexible tube. A method according to embodiments of the present invention may also comprise providing at least one microfluidic channel e.g. used to transport nutrients and/or at least one storage for nutrients. It may also comprise a micropumping system that is connected to the microfluidic channels to deliver small amounts of fluid to and from the chamber.

In a third aspect, the present invention relates to the use of bio-hybrid implants according to embodiments of the present invention, as set out with respect to embodiments of the first aspect.

Other variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

The foregoing description details certain embodiments of the invention. It will be appreciated, however, that no matter how detailed the foregoing appears in text, the invention may be practiced in many ways, and is therefore not limited to the embodiments disclosed. It should be noted that the use of particular terminology when describing certain features or aspects of the invention should not be taken to imply that the terminology is being re-defined herein to be restricted to include any specific characteristics of the features or aspects of the invention with which that terminology is associated.

## Claims

1. A bio-hybrid implant suitable for recording and/or stimulating cells, the implant comprising
- at least one closed insulated chamber (1, A, B) containing a substrate (502) with a neural interface (13) adapted for connecting neurons to an electronic circuit,
- at least one flexible guiding channel (10) having a first interface (11) to connect to at least one of the insulated chambers (1, A) and a second interface (9) to connect to a host's nerve system (7) or to another insulated chamber (B).

2. An implant according to claim 1, wherein said at least one closed insulated chamber (1, A, B) comprises at least one of an electronic circuit, a chip, a biosensor, an optical sensor, an optical stimulator, a chemical sensor, or a chemical stimulator.

3. An implant according to any of the foregoing claims, wherein the at least one closed insulated chamber (1, A, B) further comprises in-vitro cultured neuron cells (4) and optionally further supporting cells such as schwann cells, oligodendrocytes and/or glial cells.

4. An implant according to any of the previous claims, wherein the at least one closed insulated chamber (1, A, B) further comprises micro-fluidic devices for viability of the cells.

5. An implant according to any of the foregoing claims, wherein the substrate (502) further comprises micro-nail electrodes which optionally comprise in-situ CMOS circuits, special micro-structures or bio-structures.

6. An implant according to any of the foregoing claims, there being a plurality of guiding channels (10), wherein the guiding channels (10) are bundled and surrounded by a further flexible tube.

7. An implant according to any of the foregoing claims, the at least one guiding channel (10) having a longitudinal surface, wherein the at least one guiding channel (10) further comprises electrodes and/or openings (201) along its longitudinal surface.

8. Use of a bio-hybrid implant according to any of the foregoing claims for controlling and/or observing a host's nervous system by contacting in vitro neuron cells of the implant with a host's nervous system.

9. Use of a bio-hybrid implant according to any of claims 1 to 7 for recording a host's neuron activity.

10. Use of a bio-hybrid implant according to any of claims 1 to 7 for controlling a host's neuron activity by stimulating the host's nervous system.

11. Use of a bio-hybrid implant according to any of claims 1 to 7 for first controlling and then recording a host's neuron activity by first stimulating (e.g. by evoking a potential) the host's nervous system and then observing the host's neuron activity.

12. Use of a bio-hybrid implant according to any of claims 1 to 7 for first recording the host's neuron activity and then controlling a host's nervous system by first observing and then stimulating the host's neuron activity (e.g. by evoking a potential) and repeating these steps (if necessary) in a closed loop.

13. A method for manufacturing an implant according to any of claims 1 to 7, the method comprising at least steps of:
- obtaining a supporting substrate (502),
- providing on said substrate (502) a neural interface (13) for connecting neurons to an electronic circuit,
- Providing a packaging around said substrate and neural interface (13) to obtain a closed chamber (1) which is isolated from an outside environment,
- providing at least one flexible guiding channel (10) going into said closed chamber (11) and making contact to said neural interface (13),
- providing means to promote growth of neurites (12) into an inner surface of said at least one guiding channel (10).

14. A method for manufacturing an implant according to claim 13, wherein said insulated chamber (1) comprises at least one of an electronic circuit, a chip, a biosensor, an optical sensor, an optical stimulator, a chemical sensor or a chemical stimulator, the method furthermore comprising the step of providing further electronic devices for interfacing with the at least one guiding channel (10) and providing micro-fluidic devices for viability of the cells.

15. A method for manufacturing an implant according to any of claims 13 or 14, further comprising the step of providing in-vitro cultured neuron cells and/or supporting cells attached to the substrate (502).
